# EUROPÄISCHE PATENTSCHRIFT

(11) **EP 3 658 061 B1**
(45) Veröffentlichungstag und Bekanntmachung des Hinweises auf die Patenterteilung: **27.10.2021**
(21) Anmeldenummer: 19761819.2
(22) Anmeldetag: 28.08.2019
(51) Int. Cl.: A61B 17/00, A61B 17/122, A61B 50/33, A61B 50/30, A61B 50/20

(54) **VERFAHREN ZUM HERSTELLEN EINES TRAYS ZUM LAGERN VON ANEURYSMENCLIPS UND TRAY ZUM LAGERN VON ANEURYSMENCLIPS**
METHOD FOR PRODUCING A TRAY FOR STORING ANEURYSM CLIPS, AND TRAY FOR STORING ANEURYSM CLIPS
PROCÉDÉ DE FABRICATION D'UN SUPPORT POUR STOCKER DES CLIPS ANÉVRISMAUX ET SUPPORT POUR STOCKER LES CLIPS ANÉVRISMAUX

(30) Priorität: 31.08.2018 DE 102018121372
(43) Veröffentlichungstag der Anmeldung: 03.06.2020
(73) Patentinhaber: Aesculap AG, 78532 Tuttlingen (DE)
(72) Erfinder: KNITTEL, Timo, 78573 Wurmlingen (DE); HUBER, Volker, 78570 Mühlheim/Stetten (DE)
(74) Vertreter: Winter, Brandl - Partnerschaft mbB
(86) Internationale Anmeldenummer: PCT/EP2019/072967
(87) Internationale Veröffentlichungsnummer: WO 2020/043778

(56) Entgegenhaltungen:
- WO-A1-2013/119850
- WO-A2-2011/003069
- FR-A1- 2 898 277
- US-A- 4 519 501

## Beschreibung

### Technisches Gebiet

Die vorliegende Erfindung bezieht sich auf ein Verfahren zum Herstellen eines zumindest in Teilen aus Metall bestehenden Trays zum Lagern von Aneurysmenclips, insbesondere auf ein Verfahren bei dem zunächst eine Platte aus Metall bereitgestellt wird, welche anschließend mit Perforationen bzw. einem Lochmuster versehen wird. Außerdem bezieht sich die vorliegende Erfindung auf ein entsprechendes Tray zum Lagern von Aneurysmenclips.

### Stand der Technik

Es sind zum einen Trays zum Lagern von Aneurysmenclips bekannt, welche aus Kunststoff bestehen. Das Problem solcher Trays ist, dass bei Kunststoff das restlose Trocknen nach dem Waschen oder Sterilisieren der Trays nur unter großem Aufwand (d.h. mit langer Trocknungszeit und/oder hohem Trocknungsenergieaufwand) möglich ist.

Zum anderen sind bereits Trays zum Lagern von Aneurysmenclips bekannt, welche aus Metall bestehen. Das Problem dieser bisherigen Trays aus Metall ist, dass deren Herstellung zu umständlich und kostenintensiv ist.

In WO 2013/119850 A1 wird ein Sterilisationstray offenbart, welches mit einem gattungsgemäßen Verfahren hergestellt wird.

In WO 2011/003069 A2 wird ein Sterilisationstray offenbart, welches derart mit Einbuchtungen versehen ist, dass keine Trennwände notwendig sind.

### Offenbarung der Erfindung

Aufgabe der vorliegenden Erfindung ist es deshalb, ein möglichst einfach sterilisierbares Tray zum Lagern von Aneurysmenclips und ein entsprechendes, möglichst einfaches Herstellungsverfahren bereitzustellen, mittels dessen ein solches Tray zum Lagern von Aneurysmenclips hergestellt werden kann.

Diese Aufgabe wird gelöst durch ein Verfahren gemäß Anspruch 1. Dabei wird zunächst eine Platte aus Metall bereitgestellt, welche anschließend mit Perforationen versehen wird. Die Platte ist vorzugsweise aus Edelstahl (z.B. Edelstahl 1.4301 bzw. X5CrNi18-10, AISI 304, V2A, SUS304). Alternativ kann die Platte auch aus einer Titanlegierung oder aus eloxiertem Aluminium bestehen. Vorzugsweise weist die Platte eine Dicke im Bereich zwischen 0,5 mm und 2,0 mm, insbesondere eine Dicke von 0,8 mm auf. Erfindungsgemäß werden die Perforationen mittels Laserschneiden erzeugt und die mit den Perforationen versehene Platte wird mittels Biegen zu einem Well- oder Trapezblech umgeformt, das zumindest eine rinnenförmige Vertiefung zum Lagern von Aneurysmenclips aufweist.

Der Vorteil des erfindungsgemäßen Verfahrens besteht darin, dass eine einfach zu sterilisierende Aufnahme für Aneurysmenclips mit lediglich drei Schritten erzeugt wird.

Die Verwendung einer Metallplatte und das Einbringen von Perforationen oder Löchern mittels Laserschneiden hat mehrere Vorteile. Zum einen lässt sich durch Laserschneiden generell das Lochungsverhältnis, d.h. das Verhältnis von Lochfläche zur Gesamtfläche, erhöhen, da dadurch eine wesentlich filigranere Lochung und ein wirksameres Lochungsmuster gefertigt werden kann, als es beispielsweise durch Stanzen möglich ist. Zum anderen lässt die Verwendung einer Metallplatte im Vergleich zu bekannten Kunststoff-Trays auch ein solch höheres Lochungsverhältnis zu, ohne Einbußen bei der Eigenstabilität des Trays fürchten zu müssen. Aufgrund des höheren Lochungsverhältnisses kann die Fläche, an der sich Flüssigkeit sammeln kann, minimiert werden.

Gemäß der Erfindung werden die Perforationen erzeugt indem Grenzflächen, welche die einzelnen Perforationen definieren, zunächst in Teilflächen unterteilt werden, alle Teilflächen aller Grenzflächen anschließend entsprechend ihrer Ausrichtung in zumindest zwei Gruppen eingeteilt werden und die zumindest zwei Gruppen der Teilflächen aller Perforationen schließlich jeweils en bloc bzw. am Stück mittels Laserschneidens erzeugt werden. Anders ausgedrückt wird jede einzelne Perforation nicht in einem zusammenhängenden Schritt erzeugt. Vielmehr werden beispielsweise bei Perforationen mit rechteckförmigen Ausnehmungen oder Schlitzen zunächst alle Kanten aller Ausnehmungen geschnitten, welche in eine erste Richtung verlaufen, und anschließend alle Kanten aller Ausnehmungen geschnitten, die in eine zur ersten Richtung senkrechte zweite Richtung verlaufen. Dadurch werden bei der Fertigung zeitaufwändige Richtungsänderungen des Lasers vermieden. Außerdem kann dieses Vorgehen auch vorteilhaft hinsichtlich der Vermeidung von lokalen wärmeverursachten Wärmeverformungen sein. Das Erzeugen der Perforationen mittels eines Lasers erzeugt Wärme, die wiederum ein Verformen der Platte hervorrufen kann. Wird jede einzelne Perforation zunächst nur teilweise geschnitten, ist der dabei lokal eingebrachte Wärmebetrag nicht so groß wie beim Schneiden einer gesamten Perforation. Inwiefern die Teilflächen der Grenzflächen gleich ausgerichtet sein müssen, kann mittels Toleranzen vorgegeben werden. Es ist somit nicht notwendig, dass die entsprechenden Teilflächen exakt gleich ausgerichtet sind.

Gemäß einem Aspekt der Erfindung kann in die zumindest eine rinnenförmige Vertiefung des Well- oder Trapezblechs zumindest eine Steckwand, vorzugsweise werkzeuglos, montiert werden, welche die rinnenförmige Vertiefung seitlich begrenzt oder unterteilt und welche, vorzugsweise werkzeuglos, von dem Well- oder Trapezblech demontierbar ist.

Die zumindest eine erfindungsgemäße rinnenförmige Vertiefung kann grundsätzlich mit oder ohne Stirnflächen ausgebildet sein. Ist sie ohne Stirnfläche ausgebildet, kann mittels werkzeuglos demontierbarer Steckwände ein Fach erzeugt werden. Ein solches Fach oder eine mit Stirnwänden ausgebildete rinnenförmige Vertiefung kann mittels zumindest einer werkzeuglos demontierbaren Steckwand unterteilt werden. Die Steckwände sind vorzugsweise so ausgebildet, dass sie zum Verbinden mit dem Well- oder Trapezblech mit einem Teil der Perforationen zusammenwirken. Beispielsweise können sie über Clipverbindungen mit entsprechenden Perforationen verbunden sein.

Gemäß einem Aspekt der Erfindung kann die Platte vor dem Erzeugen der Perforationen an zwei gegenüberliegenden Enden eingespannt werden. Anders ausgedrückt kann die Platte in einem eingespannten Zustand perforiert werden. Durch das Einspannen kann den durch Wärme erzeugten Verformungen entgegengewirkt werden.

Ein erfindungsgemäßes Tray zum Lagern von Aneurysmenclips ist in Anspruch 4 definiert und weist ein mit lasergeschnittenen Perforationen versehenes Well- oder Trapezblech mit zumindest einer rinnenförmigen Vertiefung auf.

Die maximale Weite der Perforationen ist kleiner als 1 mm, vorzugsweise liegt sie in einem Bereich von 0,3 mm bis 1,0 mm, insbesondere in einem Bereich von 0,5 mm bis 0,8 mm. Dadurch wird sichergestellt, dass das Tray einerseits leicht und gut mit Waschflüssigkeit und/oder Sterilisierfluid (beispielsweise Wasserdampf) durchspülbar ist und andererseits die Aneurysmenclips weder durch die Perforationen fallen, noch darin steckenbleiben oder sich verheddern bzw. verkeilen oder verklemmen können.

Die maximale Stegbreite bzw. der Abstand zwischen benachbarten Perforationen ist kleiner als 0,8 mm, vorzugsweise liegt sie in einem Bereich von 0,3 mm bis 0,8 mm, insbesondere in einem Bereich von 0,5 mm bis 0,7 mm. Dadurch wird sichergestellt, dass das Tray bei ausreichender Formstabilität wenig Fläche zum Ansammeln von Flüssigkeit bietet.

Gemäß einem Aspekt der Erfindung kann das Tray zumindest eine mit dem Well- oder Trapezblech verbundene und werkzeuglos demontierbare Steckwand aufweisen, welche die rinnenförmige Vertiefung begrenzt oder unterteilt. Durch die werkzeuglose Demontierbarkeit kann die Größe einer Aufnahme für Aneurysmenclips einfach manuell variiert werden.

Gemäß einem Aspekt der Erfindung kann die zumindest eine Steckwand zumindest einen Vorsprung aufweisen und können bestimmte Perforationen derart auf die Form dieses zumindest einen Vorsprungs abgestimmt sein, dass der zumindest eine Vorsprung nur in diese bestimmten Perforationen gesteckt werden kann. Somit kann sichergestellt werden, dass nur bestimmte Größen, beispielsweise genormte Größen, für die Aneurysmenclipsaufnahme zugelassen werden.

Gemäß einem Aspekt der Erfindung können zumindest bestimmte Perforationen gleich groß und jeweils quaderförmig ausgebildet sein. "Quaderförmig" heißt in diesem Zusammenhang, dass die Perforationen eine im Wesentlichen rechteckige Kontur aufweisen. Diese quaderförmigen Perforationen können in zueinander parallelen Reihen angeordnet und mit ihrer jeweiligen Längsachse parallel zur Erstreckungsrichtung der Reihen ausgerichtet sein. Jede Reihe kann gegenüber zumindest einer angrenzenden Reihe in Erstreckungsrichtung der Reihen um eine halbe Perforationslänge versetzt sein. Anders ausgedrückt können die gleichförmig quaderförmig ausgebildeten Perforationen wie Ziegel eines mittleren Läuferverbandes eines Mauerwerks angeordnet sein. Im Gegensatz zu einer schachbrettartigen Anordnung hat die versetzte Verteilung der Perforationen den Vorteil, dass diese ein Aufweiten einzelner Perforationen erschwert.

Gemäß einem Aspekt der Erfindung kann das Tray zumindest eine mit dem Well- oder Trapezblech verbundene, werkzeuglos demontierbare Aufnahmeabdeckung aufweisen, welche die rinnenförmige Vertiefung zumindest abschnittsweise bedeckt. Mittels einer solchen Aufnahmeabdeckung kann die Aufnahme für Aneurysmenclips nach außen hin so geschlossen werden, dass ein in der Aufnahme untergebrachter Aneurysmenclip in der Aufnahme gesichert ist. Außerdem können in vorteilhafterweise Weise Informationen über den entsprechenden Aneurysmenclip auf der Außenseite der Aufnahmeabdeckung angebracht werden.

### Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Fig. 1 eine Draufsicht einer metallenen Platte;
Fig. 2 eine Draufsicht der in Fig. 2 gezeigten Platte, bei welcher ein Teil einer ersten Gruppe an Grenzflächen von Perforationen geschnitten ist;
Fig. 3 eine Draufsicht der in den Figuren 1 und 2 gezeigten Platte, bei welcher die gesamte erste Gruppe an Grenzflächen von den Perforationen geschnitten ist;
Fig. 4 eine Draufsicht der in den Figuren 1 bis 3 gezeigten Platte, bei welcher ein Teil einer zweiten Gruppe an Grenzflächen von den Perforationen geschnitten ist;
Fig. 5 eine Draufsicht der in den Figuren 1 bis 4 gezeigten Platte, bei welcher alle Grenzflächen von den Perforationen geschnitten sind;
Fig. 6 eine perspektivische Ansicht der in Figur 5 gezeigten perforierten Platte;
Fig. 7 eine perspektivische Ansicht der in Figur 6 gezeigten Platte; welche durch Biegen mit zwei rinnenförmigen Vertiefungen versehen worden ist;
Fig. 8 eine perspektivische Ansicht der in Figur 6 gezeigten Platte; welche durch Biegen zu einem Trapezblech umgeformt worden ist;
Figuren 9 und 10 perspektivische Ansichten des in Figur 8 gezeigten Trapezblechs mit Steckwänden und Stirnwänden;
Fig. 11 eine perspektivische Ansicht eines alternativ ausgebildeten Trapezblechs mit Steckwänden, Stirnwänden;
Fig. 12 eine perspektivische Ansicht des in Figur 11 gezeigten Trapezblechs mit Kennzeichnungsschildern;
Fig. 13 eine perspektivische Ansicht eines erfindungsgemäßen Trays mit offenem Deckel;
Fig. 14 eine perspektivische Ansicht des erfindungsgemäßen Trays mit geschlossenem Deckel;
Figur 15 eine perspektivische Ansicht einer weiteren Ausführungsform des erfindungsgemäßen Trays mit geschlossenem Deckel;
Figur 16 eine perspektivische Ansicht der in Fig. 15 gezeigten Ausführungsform des erfindungsgemäßen Trays mit offenem Deckel;
Fig. 17 perspektivische Ansichten eines kurzen Kennzeichnungsschilds; und
Fig. 18 perspektivische Ansichten eines langen Kennzeichnungsschilds.

Gleiche oder funktional äquivalente Merkmale sind in den einzelnen Figuren mit denselben Bezugszeichen versehen.

### Detaillierte Beschreibung bevorzugter Ausführungsformen

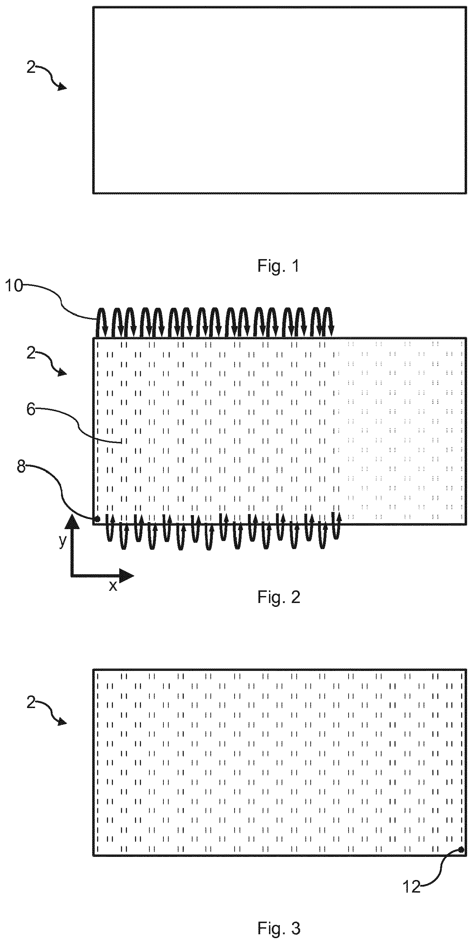

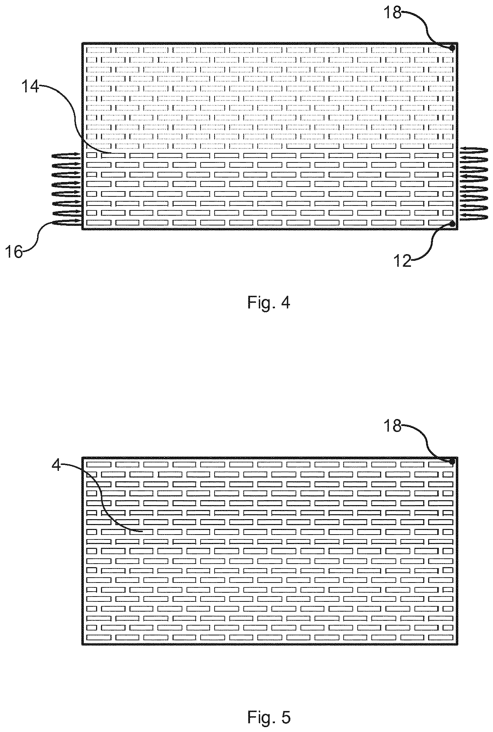

Fig. 1 zeigt eine Draufsicht einer metallenen Platte 2, welche einen im Wesentlichen rechteckigen Umriss aufweist. Um die Platte 2 mit Perforationen 4 (siehe Fig. 5) zu versehen, werden mittels eines (nicht gezeigten) Lasers zunächst erste Kanten bzw. Grenzflächen 6 der Perforationen 4 in die Platte 2 geschnitten (siehe Figuren 2 und 3). Gemeinsames Merkmal der ersten Grenzflächen 6 ist, dass sie sich alle in einer Richtung y erstrecken. Um die ersten Grenzflächen 6 möglichst effizient zu schneiden, wird an einem Startpunkt 8 in einer Ecke der Platte 2 mit dem Laserschneiden begonnen und werden die einzelnen ersten Grenzflächen 6 durch gepulstes Ansteuern des Lasers beim serpentinenhaften Abfahren der Platte 2 (siehe Pfeile 10) mit dem Laser geschnitten.

Nachdem alle ersten Grenzflächen 6 geschnitten sind und sich der Laser am Zwischenpunkt 12 befindet, werden zweite Kanten bzw. Grenzflächen 14 geschnitten. Gemeinsames Merkmal der zweiten Grenzflächen 14 ist, dass sie sich alle in einer zur Richtung y senkrechten Richtung x erstrecken. Ausgehend vom Zwischenpunkt 12, der sich ebenfalls in einer Ecke der Platte 2 befindet, werden die einzelnen zweiten Grenzflächen 14 ebenfalls durch gepulstes Ansteuern des Lasers beim serpentinenhaften Abfahren der Platte 2 (siehe Pfeile 16) mit dem Laser geschnitten.

Gepulstes Ansteuern des Lasers bedeutet hierbei, dass der Laser dann, wenn er eine Perforationslinie schneidet, im Wesentlichen bei hoher Leistung läuft. Hohe Leistung bedeutet, dass die Leistung so hoch ist, dass das zu schneidende Material bei der entsprechenden Schneidegeschwindigkeit, die der Vorschubgeschwindigkeit des Lasers entspricht, über die komplette Dicke des Materials geschnitten wird. Wird der Laser zwischen zwei Perforationslinien bewegt, wird der Laser nicht vollständig ausgeschaltet, sondern die Leistung des Lasers wird lediglich reduziert, und zwar so weit, dass die Oberfläche des Blechs nicht beschädigt wird. Dadurch, dass der Laser zwischen zwei Schneidvorgängen von zwei Perforationslinien nicht vollständig abgeschaltet wird, wird die Zeit verkürzt, bis er wieder bei hoher Leistung läuft. Auf diese Weise kann die Schneidegeschwindigkeit bzw. die Vorschubgeschwindigkeit des Lasers deutlich gegenüber dem Fall erhöht werden, in dem der Laser stets zwischen zwei Schneidvorgängen vollständig abgeschaltet wird.

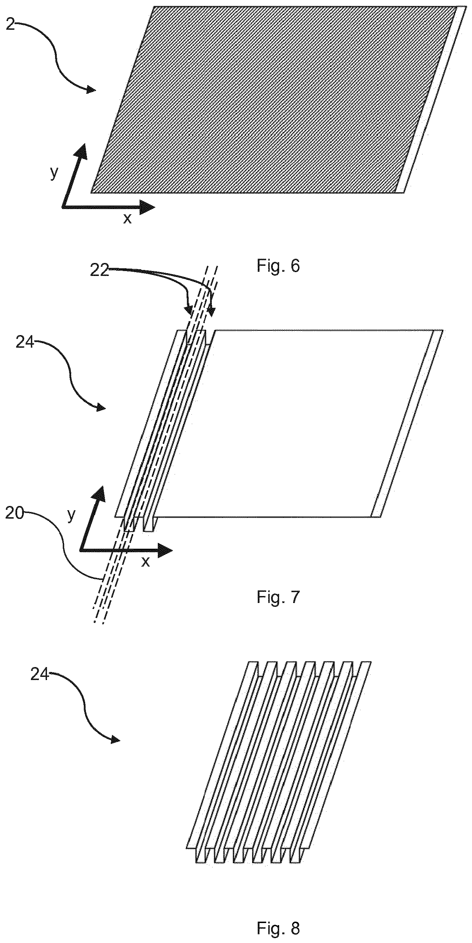

Nachdem alle zweiten Grenzflächen 14 geschnitten sind und sich der Laser am Endpunkt 18 befindet, ist das Laserschneiden der Perforationen 4 abgeschlossen (siehe Figuren 5 und 6).

Gemäß dem in den Figuren 2 bis 5 gezeigten Verfahren werden von den Perforationen 4 mit jeweils im Wesentlichen rechteckiger Kontur zunächst alle kurzen Seiten geschnitten, bevor die entsprechenden langen Seiten geschnitten werden. Alternativ ist es auch möglich, dass zunächst alle langen Seiten geschnitten werden, bevor alle kurzen Seiten geschnitten werden.

Wie in Figur 6 gezeigt, können nur Teilbereiche der Platte 2 perforiert werden (sieh schraffierten Teil der Platte 2). Beispielsweise kann ein Rand frei von Perforationen gelassen werden.

In den Figuren 7 bis 14 werden aus Gründen der Anschaulichkeit keine Perforationen gargestellt.

Die mit Perforationen 4 versehene Platte 2 wird nach dem Laserschneiden derart mehrmals um zur Richtung y parallele Kanten 20 gebogen, dass eine rinnenförmige Vertiefung 22 entsteht (siehe Fig. 7). Dieser Vorgang wird so oft wiederholt, bis die gesamte Platte 2 über ihre gesamte Fläche rinnenförmige Vertiefungen 22 aufweist (siehe Fig. 8). Die mit den rinnenförmigen Vertiefungen 22 versehene Platte wird nachfolgend Trapezblech 24 genannt.

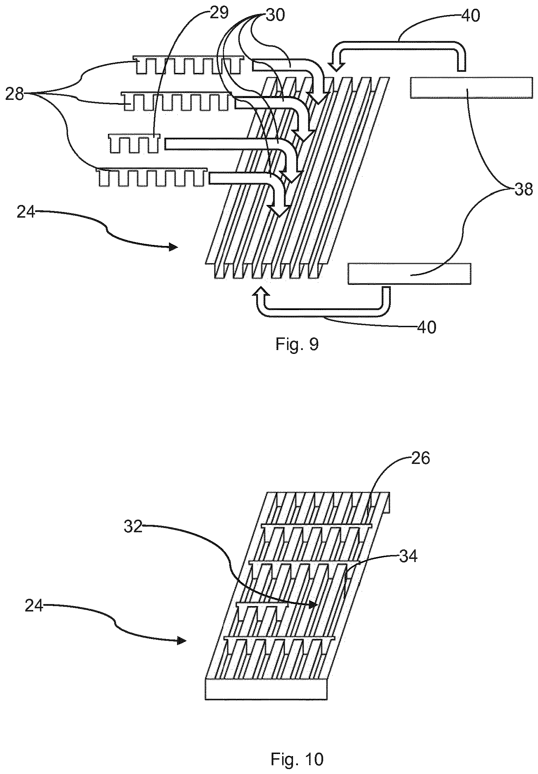

Um definierte Aneurysmenclipsaufnahmen 26 zu erzeugen, werden in die rinnenförmigen Vertiefungen 22 Steckwände 28 bzw. 29 gesteckt (siehe Pfeil 30 in Fig. 9). Für größere Aneurysmenclips werden in einem Teil 32 des Trapezblechs 24 große Aneurysmenclipsaufnahmen 34 vorgesehen. Die Steckwände 28 können massive Platten sein oder können ebenfalls Perforationen aufweisen.

Die Steckwände 28 bzw. 29 können zinnenförmig ausgebildet sein. Das heißt, dass die Steckwände 28 bzw. 29 so ausgebildet sind, dass Teilbereiche in einzelne Vertiefungen 22 des Trapezblechs 24 eintauchen und dass andere Teilbereiche der Steckwände 28 bzw. 29 die in verschiedene Vertiefungen 22 eintauchenden Teilbereiche verbinden. Die Steckwände 28 sind länger ausgebildet als die Steckwand 29, das heißt, dass eine jeweilige Steckwand 28 sechs Zinnen aufweist und alle sechs Vertiefungen 22 des Trapezblechs 24 unterteilt wohingegen die kurze Steckwand 29 nur drei Zinnen aufweist und nur drei der sechs Vertiefungen 22 unterteilt. Durch Vorsehen der kurzen Steckwand 29 können in den von der Steckwand 29 nicht unterteilten Vertiefungen die großen Aneurysmenclipsaufnahmen 34 gebildet werden.

Um Enden der rinnenförmigen Vertiefungen 22 zu verschließen, sind Stirnwände 38 vorgesehen, welche an den wellenförmigen Rändern des Trapezblechs 24 angebracht sind (siehe Pfeile 40 in Fig. 9). Vorzugsweise werden die Stirnwände 38 durch Laserschweißen mit dem Trapezblech 24 verbunden.

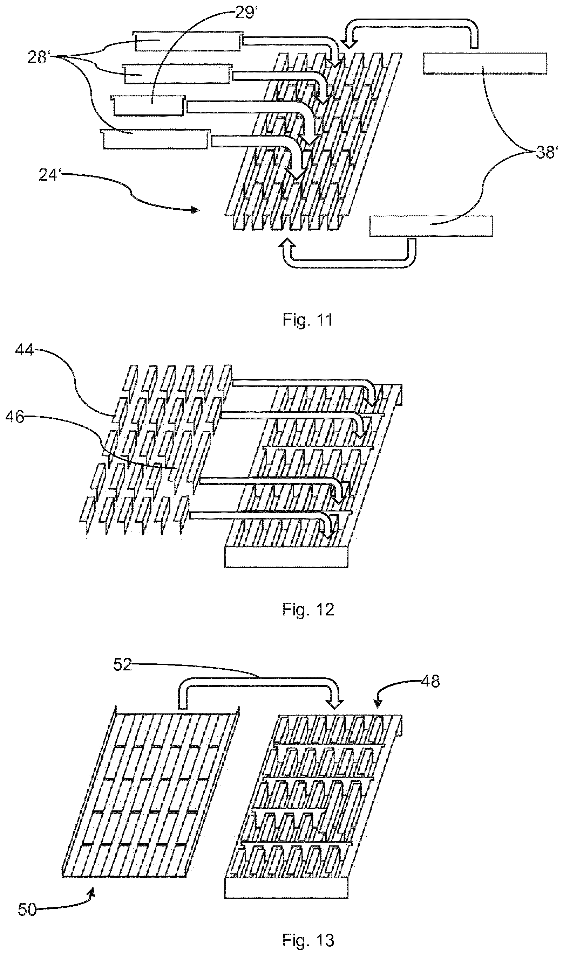

Anstatt zinnenförmige Steckwände 28 bzw. 29 vorzusehen, ist es auch möglich, das Trapezblech aus einer geschlitzten Platte herzustellen. Das entsprechend resultierende in den Figuren 11 bis 13 gezeigte Trapezblech 24' weist Schlitze 42 auf, in welche im Wesentlichen rechteckförmige Steckwände 28' bzw. 29' gesteckt werden können.

Es ist auch möglich in dem Trapezblech 24 Schlitze vorzusehen, die lediglich die Befestigung von zinnnenförmigen Steckwänden 28' bzw. 29' erleichtern bzw. verbessern.

Um die Aneurysmenclipsaufnahmen 26 und 34 beschriften zu können, sind Kennzeichnungsschilder 44 bzw. 46 vorgesehen. Zur Montage der Kennzeichnungsschilder 44 bzw. 46 werden diese von oben auf das Trapezblech 24 gesteckt (siehe Pfeil 48 in Fig. 12). Die Kennzeichnungsschilder 46 sind lang ausgebildet, das heißt, dass diese sich nur zur Beschriftung von großen Aneurysmenclipsaufnahmen 34 eignen. Die Kennzeichnungsschilder 44 sind kurz ausgebildet und dienen der Beschriftung von normalen Aneurysmenclipsaufnahmen 26. Die Kennzeichnungsschilder 44 bzw. 46 sind im Wesentlichen L-Profile. Sind die Kennzeichnungsschilder 44 bzw. 46 an einer entsprechenden Aneurysmenclipsaufnahme 24 bzw. 36 angebracht, erstreckt sich ein Schenkel des L-Profils entlang einer Seitenwand einer Aneurysmenclipsaufnahme und erstreckt sich der andere Schenkel des L-Profils in der Ebene der Zugriffsöffnung der Aneurysmenclipsaufnahme 24 bzw. 26 neben der Zugriffsöffnung. Auf der Oberseite eines jeweiligen Beschrifutngsschilds kann eine (nicht gezeigte) Abbildung des in der jeweiligen Aneurysmenclipsaufnahme 26 bzw. 34 zu lagernden Aneurysmenclips vorgesehen sein.

Wie in Fig. 13 gezeigt, bilden das Trapezblech 24, die Stirnwände 38, die Steckwände 28 bzw. 29 und die Kennzeichnungsschilder 44 bzw. 46 ein erfindungsgemäßes Tray 48.

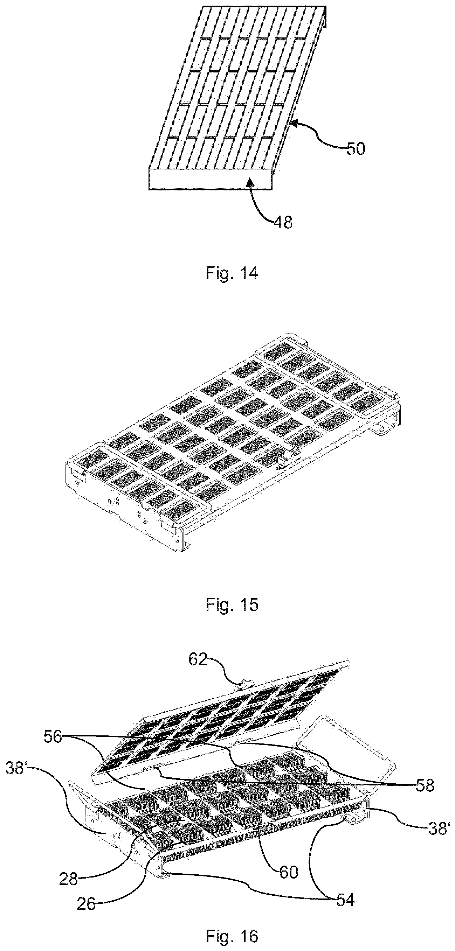

Wie in den Figuren 13 und 14 gezeigt, kann das Tray 48 mit einem Deckel 50 geschlossen werden (siehe Pfeil 52 in Fig. 13). Vorzugsweise wird das Tray 48 aus einem Blech mit einer Dicke von 0,8 mm hergestellt und wird der Deckel 50 aus einem Blech mit der Dicke von 1 mm hergestellt.

Die in den Figuren 13 und 14 gezeigte und oben beschriebene Ausführungsform des erfindungsgemäßen Trays zum Lagern von Aneurysmenclips stellt lediglich eine mögliche Umsetzung der beanspruchten Erfindung dar.

Gemäß einer anderen in den Figuren 15 und 16 gezeigten vorteilhaften Ausführungsform wird das geschnittene Lochblech der Fig. 6 in einzelne Streifen geschnitten und aus jedem Streifen wird dann eine rinnenförmige Vertiefung 22 mit angrenzender Aufnahmefläche für einen Kennzeichnungsschild gebogen. Mehrere dieser so ausgebildeten Elemente werden mittels Laserschweißen zu einer Gruppe zusammengebaut. An die Stirnseiten der rinnenförmigen Vertiefungen 22 werden Endbleche bzw. Stirnwände 38' angeschweißt und so ein Tray 24'" gebildet. Die Stirnwände 38' verfügen über Standfüße 54 und Aufnahmen 56, in die Vorsprünge 58 eines Deckels 50' eingeschoben werden können, sowie eine Aufnahme 60 für ein Verschlusselement 62, welches an dem Deckel 50' vorgesehen ist und dazu dient, den Deckel 50' an dem Tray 24" lösbar zu befestigen und dabei die rinnenförmigen Vertiefungen 22 abzudecken und so zu verschließen. Steckwände 28 können quer zu den rinnenförmigen Vertiefungen 22 lösbar eingebracht werden, um aus den rinnenförmigen Vertiefungen 22 mehrere getrennte Aneurysmenclipsaufnahmen 26 für unterschiedliche Aneurysmenclips auszubilden. Vorzugsweise sind die Aufnahmeflächen so geschlitzt, dass die Steckwände 28 von oben bis zum Boden der rinnenförmigen Vertiefungen 22 eingeschoben werden können.

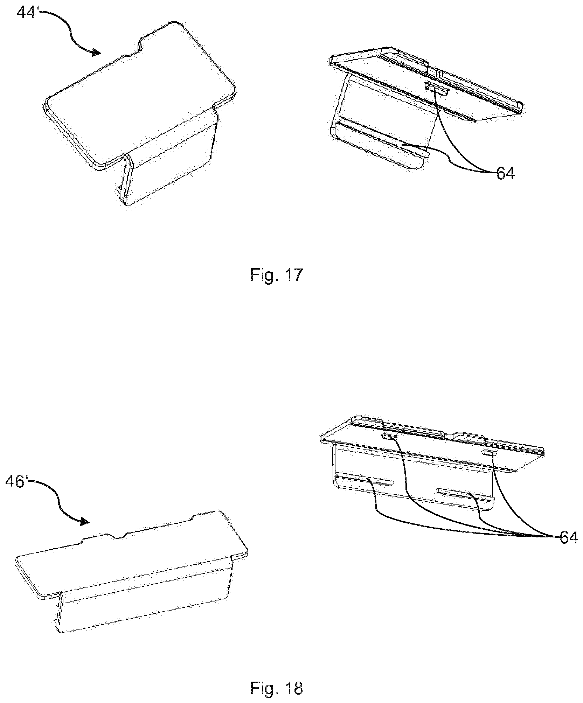

In den Figuren 17 und 18 sind kurze und lange Kennzeichnungsschilder 64 bzw. 66 gezeigt. Um mit dem entsprechenden in Figur 16 gezeigten Trapezblech 24" verbindbar zu sein, weisen die als L-Profile ausgebildeten Kennzeichnungsschilder 64 bzw. 66 an ihren Schenkeln jeweils zumindest einen Vorsprung 68 auf, welche in entsprechende Ausnehmungen im Trapezblech 24" eingeclipst werden können.

### Bezugszeichenliste

- 2: metallene Platte
- 4: Perforationen
- 6: erste Grenzflächen
- 8: Startpunkt des Lasers
- 10: Bewegung des Lasers beim Schneiden der ersten Grenzflächen
- 12: Zwischenpunkt des Lasers nach Schneiden aller ersten Grenzflächen
- 14: zweite Grenzflächen
- 16: Bewegung des Lasers beim Schneiden der zweiten Grenzflächen
- 18: Endpunkt des Lasers nach Schneiden aller zweiten Grenzflächen
- 20: Biegekanten
- 22: rinnenförmige Vertiefungen
- 24, 24', 24": Trapezblech
- 26: (normale) Aneurysmenclipsaufnahme
- 28; 28'; 29; 29': Steckwand
- 30: Bewegung beim Montieren einer Steckwand
- 32: Teil des Trapezblechs mit großen Aneurysmenclipsaufnahmen
- 34: große Aneurysmenclipsaufnahme
- 36: Aufnahmeabdeckung für normale Aneurysmenclipsaufnahme
- 38,38': Stirnwand
- 40: Bewegung beim Montieren der Stirnwand
- 42: Schlitz
- 44: (normales) Kennzeichnungsschild
- 46: großes Kennzeichnungsschild
- 48: Tray
- 50: Deckel
- 52: Bewegung beim Verschließen des Deckels
- 54: Standfuß
- 56,60: Aufnahme
- 58; 64: Vorsprung
- 62: Verschlusselement

## Patentansprüche

1. Verfahren zum Herstellen eines Trays (44) zum Lagern von Aneurysmenclips, bei dem zunächst eine metallene Platte (2) bereitgestellt wird, welche anschließend mit Perforationen (4) versehen wird,
die Perforationen (4) mittels Laserschneidens erzeugt werden, und
die mit den Perforationen (4) versehene Platte (2) mittels Biegens zu einem Well- oder Trapezblech (24) umgeformt wird, das zumindest eine rinnenförmige Vertiefung (22) zum Lagern von Aneurysmenclips aufweist,
**dadurch gekennzeichnet, dass**
die Perforationen (4) erzeugt werden, indem Grenzflächen (6, 14), welche die einzelnen Perforationen (4) definieren, zunächst in Teilflächen unterteilt werden, alle Teilflächen aller Grenzflächen anschließend entsprechend ihrer Ausrichtung in zumindest zwei Gruppen eingeteilt werden und die zumindest zwei Gruppen der Teilflächen schließlich jeweils en bloc mittels Laserschneidens erzeugt werden.

2. Verfahren nach Anspruch 1, **dadurch gekennzeichnet, dass** in die zumindest eine rinnenförmige Vertiefung (22) des Well- oder Trapezblechs (24) zumindest eine Steckwand (28, 29), vorzugsweise werkzeuglos, montiert wird, welche die rinnenförmige Vertiefung (22) seitlich begrenzt oder unterteilt und welche, vorzugsweise werkzeuglos, von dem Well- oder Trapezblech (24) demontierbar ist.

3. Verfahren nach Anspruch 1 oder 2, **dadurch gekennzeichnet, dass** die Platte (2) vor dem Erzeugen der Perforationen (4) an zwei gegenüberliegenden Enden eingespannt wird.

4. Tray (44) zum Lagern von Aneurysmenclips, welches insbesondere nach dem Verfahren nach einem der Ansprüche 1 bis 3 hergestellt ist und ein mit lasergeschnittenen Perforationen (4) versehenes Well- oder Trapezblech (24) mit zumindest einer rinnenförmigen Vertiefung (22) aufweist,
**dadurch gekennzeichnet, dass**
die maximale Weite der Perforationen kleiner als 1 mm ist, vorzugsweise in einem Bereich von 0,3 mm bis 1,0 mm liegt, insbesondere in einem Bereich von 0,5 mm bis 0,8 mm liegt; und/oder
die maximale Stegbreite zwischen benachbarten Perforationen kleiner als 0,8 mm ist, vorzugsweise in einem Bereich von 0,3 mm bis 0,8 mm liegt, insbesondere in einem Bereich von 0,5 mm bis 0,7 mm liegt.

5. Tray (44) nach Anspruch 4, **gekennzeichnet durch** zumindest eine mit dem Well- oder Trapezblech (24) werkzeuglos montierbare und demontierbare Steckwand (28; 29), welche die rinnenförmige Vertiefung (22) seitlich begrenzt oder unterteilt.

6. Tray (44) nach Anspruch 5, **dadurch gekennzeichnet, dass** bestimmte Perforationen (4) jeweils eine auf zumindest einen Vorsprung der zumindest einen Steckwand (28; 29) abgestimmte Kontur aufweisen, so dass die zumindest eine Steckwand (28; 29) nur durch Zusammenwirken ihres zumindest einen Vorsprungs mit einer der auf den Vorsprung abstimmten Perforationen (4) mit dem Well- oder Trapezblech (24) verbunden werden kann.

7. Tray (44) nach einem der Ansprüche 4 bis 6, **dadurch gekennzeichnet, dass** zumindest bestimmte Perforationen (4) gleich groß und jeweils rechteckförmig ausgebildet sind, diese rechteckförmigen Perforationen (4) in zueinander parallelen Reihen angeordnet und mit ihrer jeweiligen Längsachse parallel zur Erstreckungsrichtung der Reihen ausgerichtet sind und jede Reihe gegenüber zumindest einer angrenzenden Reihe in Erstreckungsrichtung der Reihen, insbesondere um eine halbe Perforationslänge, versetzt ist.

8. Tray (44) nach einem der Ansprüche 4 bis 7, **gekennzeichnet durch** zumindest eine mit dem Well- oder Trapezblech (24) verbundene, werkzeuglos demontierbare Aufnahmeabdeckung (36, 38), welche die rinnenförmigen Vertiefung zumindest abschnittsweise bedeckt.

## Claims

1. A method for producing a tray (44) for storing aneurism clips, in which first a metal plate (2) is provided, which is subsequently provided with perforations (4),
the perforations (4) are generated by laser cutting, and
the plate (2) provided with the perforations (4) is formed via bending into a corrugated or trapezoidal sheet (24) which has at least one channel-shaped indentation (22) for storing aneurism clips,
**characterized in that**
the perforations (4) are generated by first subdividing border surfaces (6, 14), which define the individual perforations (4), into partial surfaces, then dividing all partial surfaces of all border surfaces into at least two groups according to their orientation, and finally generating the at least two groups of partial surfaces en bloc in each case by laser cutting.

2. The method according to claim 1, **characterized in that** at least one plug-in wall (28, 29) is mounted in the at least one channel-shaped indentation (22) of the corrugated or trapezoidal sheet (24), preferably without tools, said plug-in wall (28, 29) laterally delimiting or subdividing the channel-shaped indentation (22) and being removable from the corrugated or trapezoidal sheet (24), preferably without tools.

3. The method according to claim 1 or 2, **characterized in that** the plate (2) is clamped at two opposite ends before generating the perforations (4).

4. A tray (44) for storing aneurism clips, which is produced in particular by the method according to one of claims 1 to 3 and comprises a corrugated or trapezoidal sheet (24) provided with laser-cut perforations (4) and having at least one channel-shaped indentation (22),
**characterized in that**
the maximum width of the perforations is smaller than 1 mm, preferably lies in a range of 0.3 mm to 1.0 mm, in particular lies in a range of 0.5 mm to 0.8 mm; and/or
the maximum web width between adjacent perforations is less than 0.8 mm, preferably in a range from 0.3 mm to 0.8 mm, in particular in a range from 0.5 mm to 0.7 mm.

5. The tray (44) according to claim 4, **characterized by** at least one plug-in wall (28; 29) which can be mounted and dismounted without tools with the corrugated or trapezoidal sheet (24) and which laterally delimits or subdivides the channel-shaped indentation (22).

6. The tray (44) according to claim 5, **characterized in that** certain perforations (4) each have a contour matched to at least one projection of the at least one plug-in wall (28; 29), so that the at least one plug-in wall (28; 29) can be connected to the corrugated or trapezoidal sheet (24) only by cooperation of its at least one projection with one of the perforations (4) matched to the projection.

7. The tray (44) according to one of claims 4 to 6, **characterized in that** at least specific perforations (4) are of the same size and are each rectangular in shape, wherein these rectangular perforations (4) are arranged in rows parallel to each other and are oriented with their respective longitudinal axes parallel to the direction in which the rows extend, and each row is offset with respect to at least one adjacent row in the direction in which the rows extend, in particular by half a perforation length.

8. The tray (44) according to one of claims 4 to 7, **characterized by** at least one receiving cover (36, 38) which is connected to the corrugated or trapezoidal sheet (24), can be removed without tools and covers the channel-shaped indentation at least in sections.

## Revendications

1. Procédé de fabrication d'un plateau (44) pour le stockage de clips d'anévrisme, dans lesquels une plaque métallique (2) est d'abord fournie, qui est ensuite pourvue de perforations (4),
les perforations (4) sont produites au moyen d'une découpe au laser, et
la plaque (2) pourvue de perforations (4) est formée par pliage en une tôle ondulée ou trapézoïdale (24), qui présente au moins un creux en forme de canal (22) pour le stockage des clips d'anévrisme,
**caractérisé en ce que**
les perforations (4) sont créées en divisant d'abord les surfaces de délimitation (6, 14) définissant les perforations individuelles (4) en surfaces partielles, toutes les surfaces partielles de toutes les surfaces de délimitation sont ensuite divisées, selon leur orientation, en au moins deux groupes, et les au moins deux groupes des surfaces partielles sont enfin produit en bloc par découpe laser.

2. Procédé selon la revendication 1, **caractérisé en ce que** au moins une paroi enfichable (28, 29) est montée, de préférence sans outils, dans le au moins un creux en forme de canal (22) de la tôle ondulée ou trapézoïdale (24), laquelle paroi délimite ou subdivise latéralement le creux en forme de canal (22) et peut être démontée, de préférence sans outils, de la tôle ondulée ou trapézoïdale (24).

3. Procédé selon la revendication 1 ou 2, **caractérisé en ce que** la plaque (2) est serrée à deux extrémités opposées avant la réalisation des perforations (4).

4. Plateau (44) pour le stockage de clips d'anévrisme, qui est fabriqué en particulier par le procédé selon l'une des revendications 1 à 3 et qui présente une tôle ondulée ou trapézoïdale (24) pourvue de perforations (4) découpées au laser et présentant au moins un creux (22) en forme de canal,
**caractérisé en ce que**
la largeur maximale des perforations est inférieure à 1 mm, de préférence dans une plage de 0,3 mm à 1,0 mm, en particulier dans une plage de 0,5 mm à 0,8 mm ; et/ou la largeur maximale de la bande entre les perforations adjacentes est inférieure à 0,8 mm, de préférence dans une plage de 0,3 mm à 0,8 mm, en particulier dans une plage de 0,5 mm à 0,7 mm.

5. Plateau (44) selon la revendication 4, **caractérisé par** au moins une paroi enfichable (28; 29) qui peut être montée et démontée sans outils avec la tôle ondulée ou trapézoïdale (24) et qui délimite ou subdivise latéralement le creux en forme de canal (22).

6. Plateau (44) selon la revendication 5, **caractérisé en ce que** chacune de certaines perforations (4) ont un contour adapté à au moins une saillie de la au moins une paroi enfichable (28 ; 29), de sorte que la au moins une paroi enfichable (28, 29) ne peut être reliée à la tôle ondulée ou trapézoïdale (24) que par interaction de sa au moins une saillie avec l'une des perforations (4) adaptées à la saillie.

7. Plateau (44) selon l'une des revendications 4 à 6, **caractérisé en ce qu'**au moins certaines perforations (4) ont la même taille et sont chacune de forme rectangulaire, ces perforations rectangulaires (4) sont disposées en rangées parallèles les unes aux autres et sont alignées avec leur axe longitudinal respectif parallèle à la direction d'extension des rangées, et chaque rangée est décalée par rapport à au moins une rangée adjacente dans la direction d'extension des rangées, en particulier d'une demi-longueur de perforation.

8. Plateau (44) selon l'une des revendications 4 à 7, **caractérisé par** un couvercle (36, 38) qui est relié à la tôle ondulée ou trapézoïdale (24) et peut être démonté sans outils et qui recouvre le creux en forme de canal au moins par sections.
